# EUROPÄISCHE PATENTSCHRIFT

(11) **EP 1 716 149 B1**
(45) Veröffentlichungstag und Bekanntmachung des Hinweises auf die Patenterteilung: **12.12.2012**
(21) Anmeldenummer: 05706514.6
(22) Anmeldetag: 18.02.2005
(51) Int. Cl.: C07D 475/04, A61K 31/522, A61P 35/00

(54) **Amorphe (6S)-Folinsäure zur Herstellung von stabilen wässrigen Lösungen von Natrium- oder Kalium (6S)-Folinat**
Amorphous (6S)-folinic acid useful for preparing stable aqueous solutions of sodium or potassium (6S)-folinate
L'acide (6S)-folinique amorphe utile pour la préparation de solutions aqueuses stables de sodium ou de potassium (6S)-folinate

(30) Priorität: 20.02.2004 CH 285042004
(43) Veröffentlichungstag der Anmeldung: 02.11.2006
(73) Patentinhaber: Cerbios-Pharma S.A., CH-6917 Barbengo (CH)
(72) Erfinder: GIANCARLO, Francese, CH-6926 Montagnola (CH); MOROSOLI, Moreno, CH-6950 Tesserete (CH)
(74) Vertreter: Zink-Wild, Markus Peter
(86) Internationale Anmeldenummer: PCT/CH2005/000092
(87) Internationale Veröffentlichungsnummer: WO 2005/080395

(56) Entgegenhaltungen:
- EP-A- 0 293 029
- EP-A- 0 401 895
- EP-A- 0 495 204
- EP-A- 0 667 159
- WO-A-93/17022
- WO-A-95/26963
- US-A- 4 148 999
- EDWIN H. FLYNN: "A Synthetic Compound with Folinic Acid Activity" JOURNAL OF THE AMERICAN CHEMICAL SOCIETY., Bd. 73, 1951, Seiten 1979-1982, XP002287843 USAMERICAN CHEMICAL SOCIETY, WASHINGTON, DC.
- BARBARA ROTH: "Synthesis of Leucovorin" JOURNAL OF THE AMERICAN CHEMICAL SOCIETY., Bd. 74, 1952, Seiten 3247-3252, XP002287844 USAMERICAN CHEMICAL SOCIETY, WASHINGTON, DC.
- TEMPLE C ET AL: "PREPARATION AND PURIFICATION OF L-(PLUS OR MINUS)-5-FORMYL-5,6,7,8-TE TRAHYDROFOLIC ACID" JOURNAL OF MEDICINAL CHEMISTRY, AMERICAN CHEMICAL SOCIETY. WASHINGTON, US, Bd. 22, Nr. 6, 1. Juni 1979 (1979-06-01), Seiten 731-734, XP002074295 ISSN: 0022-2623
- FEENEY ET AL.: "Hydrogen-1 Nuclear Magnetic Resonance Study of the Complexes of Two Diastereoisomers of Folinic Acid with Dihydrofolate Reductase" BIOCHEMISTRY., Bd. 20, 1981, Seiten 1837-1842, XP002352951 USAMERICAN CHEMICAL SOCIETY. EASTON, PA.
- KHALIFA ET AL: HELVETICA CHIMICA ACTA., Bd. 63, 1980, Seiten 2554-2558, XP002352952 CHVERLAG HELVETICA CHIMICA ACTA. BASEL. in der Anmeldung erwähnt

## Beschreibung

Die vorliegende Erfindung betrifft ein Verfahren zur Herstellung von amorpher (6S)-N(5)-Formyl-5,6,7,8-tetrahydrofolsäure.

Die vorliegende Erfindung betrifft auch amorphe (6S)-N(5)-Formyl-5,6,7,8-tetrahydrofolsäure als solche.

Die vorliegende Erfindung betrifft auch ein Verfahren zur Herstellung einer konzentrierten, stabilen Lösung, insbesondere einer Injektionslösung oder Infusionslösung, des Natrium- oder Kaliumsalzes von (6RS)- oder (6S)-Folinsäure.

N(5)-Formyl-5,6,7,8-tetrahydrofolsäure wird auch Folinsäure genannt.

Die pharmakologische Bedeutung von den gut löslichen Alkalimetallsalzen von reduzierten Folaten ist in der Beschreibungseinleitung von EP 0 667 159 beschrieben.

Der bekannte Stand der Technik zur Herstellung von Folinsäure ist in NO 172 492 beschrieben.

Mit diesen Hinweisen werden die in diesen beiden Literaturstellen gemachten Aussagen auch als hierin offenbart betrachtet.

Es ist davon auszugehen, dass die gemäss diesen Verfahren hergestellte Folinsäure in amorpher Form vorliegt.

Kristalline Folinsäure wurde bis zum Anmeldedatum der vorliegenden Erfindung noch nicht beschrieben.

In Beispiel 1 von WO 93/17022 wird die Herstellung von reiner (6RS)-Folinsäure beschrieben. In diesem Beispiel wird eine "Fällung" ("precipitate") von (6RS)-Folinsäure erwähnt.

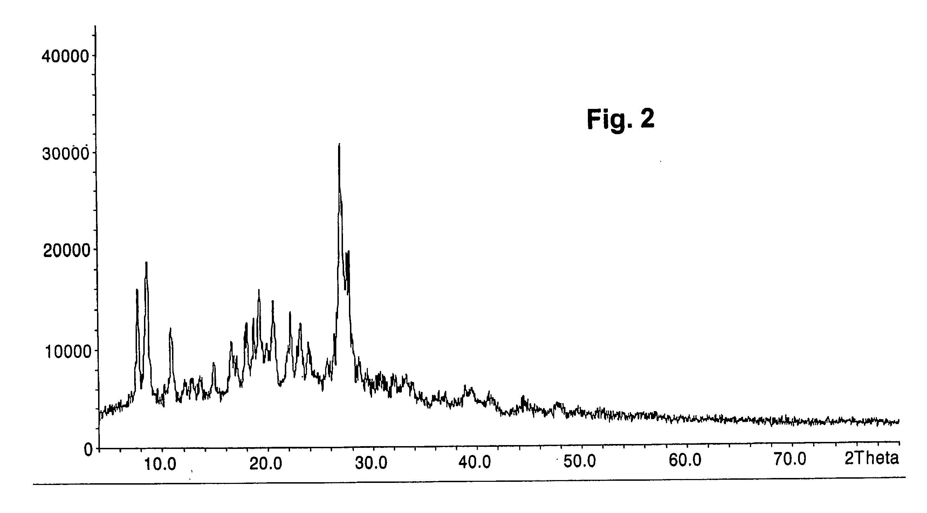

Aus dem in Figur 2 gezeigten Pulverröntgendiagramm des gemäss diesem Beispiel erhaltenen Produktes ist ersichtlich, dass dieses Produkt mindestens 30 % an amorpher (6RS)-Folinsäure enthält.

Man würde erwarten, dass man Folinsäure durch direktes Ansäuern einer wässrigen Lösung eines wasserlöslichen Folinatsalzes erhalten würde.

Wenn man beispielsweise eine wässrige Lösung von Calcium-Folinat mit verdünnter Salzsäure ansäuert, dann erhält man ein unbehandelbares, gummiartiges Produkt, und zwar selbst dann, wenn verschiedene Parameter, wie Temperatur, Konzentration, Reaktionszeit, variiert werden.

Gemäss Beispiel 6 von EP 0 293 029 wird eine wässrige Lösung von Calcium-(6S)-Folinat vorsichtig mit verdünnter Salzsäure versetzt, wobei die (6S)-Folinsäure ausfallen und durch Filtration gewonnen werden sollte.

Die Anmelderin der vorliegenden Erfindung konnte dieses Ausführungsbeispiel nicht wiederholen: es wurde jeweils ein unbehandelbares, gummiartiges Produkt erhalten, obwohl verschiedene Parameter, wie Temperatur, Konzentration, Reaktionszeit, variiert wurden.

Gemäss E. Khalifa, A. N. Ganguly, J. H. Bieri und M. Viscontini, Helv. Chim. Acta, Vol. 63, 2554 (1980) liegt die hierin beschriebene Folinsäure eindeutig als (6RS)-Diastereoisomerengemisch und in amorpher Form vor.

Demnach ist im Ausführungsbeispiel, welches in EP 0 667 159 beschrieben ist, amorphe (6RS)-Folinsäure verwendet worden.

Es ist allgemein bekannt, dass die biologisch aktive Form der reduzierten Folate die (6S)-Konfiguration hat; siehe beispielsweise E. E. van Tamelen, R. E. Hopla, Journal of the American Chemical Society, 101, 6114-6115, (1979).

Daraus folgt, dass die in EP 0 667 159 beschriebene Injektionslösung 50 % an inaktiver Substanz mit der (6R)-Konfiguration enthält. Das wiederum bedeutet, dass der menschliche Körper, dem eine solche Injektionslösung verabreicht wird, unnötig belastet wird (doppelte Dauer der Verabreichung und Verabreichung einer inaktiven Substanz).

Es ist ein Ziel der vorliegenden Erfindung, die oben genannten Nachteile zu überwinden.

Es ist ein weiteres Ziel der vorliegenden Erfindung, amorphe (6S)-Folinsäure zur Verfügung zu stellen.

Ebenso soll ein Verfahren zur Herstellung dieser Verbindung zur Verfügung gestellt werden.

Es soll auch eine konzentrierte, stabile Lösung des Natrium- oder Kaliumsalzes von (6S)-Folinsäure zur Verfügung gestellt werden.

Es soll auch ein Verfahren zur Herstellung dieser Lösung, ausgehend von amorpher (6S)-Folinsäure, zur Verfügung gestellt werden.

Mit der vorliegenden Erfindung werden diese Ziele erreicht.

Es wurde auch gefunden, dass die gemäss der Lehre des kennzeichnenden Teiles von Anspruch 1 hergestellte amorphe (6S)-Folinsäure eine mit der kristallinen (6RS)-Folinsäure vergleichbare Stabilität hat.

Das erfindungsgemässe Verfahren zur Herstellung von amorpher (6S)-N(5)-Formyl-5,6,7,8-tetrahydrofolsäure,
ist dadurch gekennzeichnet, dass man zu gerührtem Wasser, welches eine Temperatur von 2°C bis 12°C hat, gleichzeitig
- eine wässrige Lösung, welche eine Temperatur von 40°C bis 50°C hat, von (6S)-Calcium-Folinat, und
- eine wässrige Lösung von Salzsäure oder Essigsäure
derart hinzugibt, dass im erhaltenen Gemisch während der Hinzugabe der beiden genannten Lösungen einerseits die Temperatur auf einem Wert von 2°C bis 12°C gehalten wird, und andererseits der pH-Wert auf einem Wert von 2,5 bis 3,5 gehalten wird,
den entstandenen Festkörper mittels Filtration oder Zentrifugation isoliert,
diesen Festkörper zuerst mit kaltem Wasser und dann mit einem wässrigen organischen Lösungsmittel wäscht, und
den gewaschenen Festkörper, nämlich amorphe (6S)-N(5)-Formyl-5,6,7,8-tetrahydrofolsäure, unter reduzierten Druck trocknet und gewinnt.

Das erfindungsgemässe Verfahren zur Herstellung einer konzentrierten, stabilen Lösung, insbesondere einer Injektionslösung oder Infusionslösung, des Natrium- oder Kaliumsalzes von (6S)-Folinsäure,

ist dadurch gekennzeichnet, dass man amorphe (6S)-Folinsäure in Wasser, welches entgast und für die Herstellung von Injektionslösungen oder Infusionslösungen annehmbar ist, bei Raumtemperatur unter einer Inertgasatmosphäre suspendiert, dann
eine wässrige Lösung von Natrium- oder Kaliumhydroxid, -hydrogencarbonat oder -carbonat portionenweise so lange hinzugibt, bis eine klare Lösung entstanden ist, welche den jeweils gewünschten pH-Wert aufweist,
die erhaltene Lösung einer sterilen Filtration unterwirft, und
die erhaltene sterile Lösung unter einer Inertgasatmosphäre in Vials oder in Ampullen abfüllt.

Die erfindungsgemässe konzentrierte, stabile Lösung, insbesondere eine Injektionslösung oder Infusionslösung, ist dadurch gekennzeichnet, dass sie nebst Wasser entweder (6S)-Natrium-Folinat oder (6S)-Kalium-Folinat enthält.

Diese erfindungsgemässe Lösung kann verwendet werden
- zur Herstellung eines Arzneimittels für Hilfeleistungen - rescue agent - nach Behandlung mit hohen Dosen an Methotrexat, oder
- zur Herstellung eines Arzneimittels, welches mit 5-Fluorouracil kombiniert wird, oder
- zur Herstellung eines Arzneimittels für die Behandlung von megaloblastischen Anämien und Dihydropteridin Reduktase Defizienz.

Bevorzugte Ausführungsformen der vorliegenden Erfindung sind in den abhängigen Ansprüchen definiert.

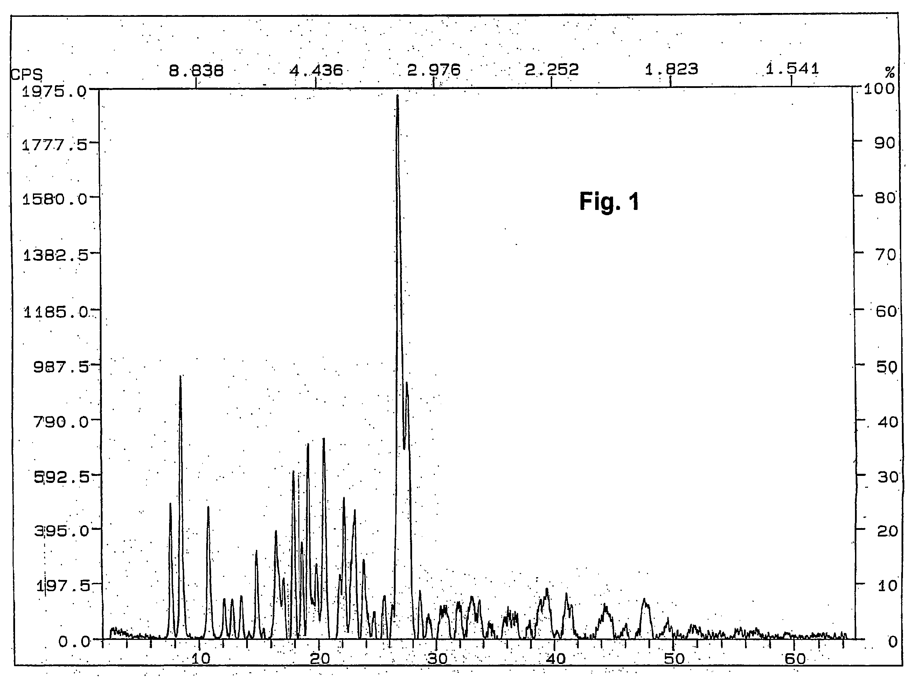

Figur 1 zeigt ein Pulverröntgendiagramm von erfindungsgemässer kristalliner (6RS)-Folinsäure.

Aus Figur 1 ist ersichtlich, dass die Verbindung (6RS)-Folinsäure hochkristallin ist.

Es wurde überraschend festgestellt, dass die gemäss der Lehre des kennzeichnenden Teiles von Anspruch 1 hergestellte (6S)-N(5)-Formyl-5,6,7,8-tetrahydrofolsäure nicht in kristalliner Form erhältlich ist. Das entsprechende Pulverröntgendiagramm zeigt keine signifikanten Ausschläge (peaks).

Die nachfolgenden Beispiele illustrieren die vorliegende Erfindung.

### Beispiel 1 (nicht erfindungsgemäβ)

250 g (6RS)-Calcium-Folinat, hergestellt gemäss NO 172 492, wurden in 3,3 Liter deionisiertem Wasser bei einer Temperatur von 55°C gelöst.

Diese klare Lösung (nachfolgend als Lösung A bezeichnet) wurde auf eine Temperatur von 50°C abgekühlt.

In ein 10 Liter Reaktorgefäss mit Kühl- und Rührvorrichtungen wurden 2,52 Liter deionisiertes Wasser gegeben und unter Rühren auf eine Temperatur von 6°C abgekühlt.

Zu diesem 6°C kalten Wasser wurden unter Rühren gleichzeitig die genannte Lösung A und 18%-ige wässrige Salzsäure mittels zwei peristaltischen Pumpen zugegeben.

Die Geschwindigkeit der Hinzugabe der Lösung A wurde so gewählt, dass die Temperatur im erhaltenen Gemisch zwischen 6°C und 10°C blieb.

Die relative Geschwindigkeit der Hinzugabe der 18%-eigen wässrigen Salzsäure wurde so gewählt, dass der pH-Wert im erhaltenen Gemisch zwischen 2,8 und 3,2 blieb.

Die Hinzugabe der Lösung A und der Salzsäure war nach 3 Stunden beendet.

Die erhaltene Suspension wurde während 1 Stunde bei einer Temperatur von 6°C und 10°C weiter gerührt.

Der entstandene kristalline Festkörper wurde mittels Zentrifugation isoliert.

Der Festkörper wurde einmal mit deionisiertem Wasser und einmal mit einem 9:1 Gemisch (v/v) von Aceton und deionisiertem Wasser gewaschen. Beide Waschlösungen hatten eine Temperatur von 5°C bis 10°C.

Der gewaschene, kristalline Festkörper wurde unter reduziertem Druck (20 bis 30 mbar) bei Raumtemperatur während 2 Stunden und bei einer Temperatur von 50°C während 1 Stunde getrocknet.

Es wurden 215 g kristalline (6RS)-Folinsäure erhalten.
HPLC-Reinheit: 99,6 %
HPLC-Assay/Gehalt: 100,3 %

Es folgt aus Figur 1, dass der erhaltene Festkörper kristallin war.

Dieser Festkörper wurde bei einer Temperatur von 2°C bis 8°C in einem Kühlschrank gelagert. Dabei wurden für diesen Festkörper die folgenden Stabilitätsdaten erhalten:

| Zeit (Monate) | Assay (HPLC) | Reinheit (HPLC) |
|---|---|---|
| 0 | 99, 4 % | 99, 78 % |
| 3 | 99, 0 % | 99, 79 % |
| 6 | 99, 1 % | 99, 75 % |
| 9 | 100, 2 % | 99, 82 % |
| 12 | 99, 9 % | 99, 79 %. |

### Beispiel 2

290 g (6S)-Calcium-Folinat, hergestellt gemäss EP 600 460 und NO 172 492, wurden in 4,9 Liter deionisiertem Wasser bei einer Temperatur von 58°C gelöst.

Diese klare Lösung (nachfolgend als Lösung B bezeichnet) wurde auf eine Temperatur von 45°C abgekühlt.

In ein 20 Liter Reaktorgefäss mit Kühl- und Rührvorrichtungen wurden 4 Liter deionisiertes Wasser gegeben und unter Rühren auf eine Temperatur von 6°C abgekühlt.

Zu diesem 6°C kalten Wasser wurden unter Rühren gleichzeitig die genannte Lösung B und 18%-ige wässrige Salzsäure mittels zwei peristaltischen Pumpen zugegeben.

Die Geschwindigkeit der Hinzugabe der Lösung B wurde so gewählt, dass die Temperatur im erhaltenen Gemisch zwischen 6°C und 10°C blieb.

Die relative Geschwindigkeit der Hinzugabe der 18%-igen wässrigen Salzsäure wurde so gewählt, dass der pH-Wert im erhaltenen Gemisch zwischen 2,8 und 3,2 blieb.

Die Hinzugabe der Lösung B und der Salzsäure war nach 3 Stunden beendet.

Die erhaltene Suspension wurde während 1 Stunde bei einer Temperatur von 6°C und 10°C weiter gerührt.

Der entstandene amorphe Festkörper wurde mittels Zentrifugation isoliert.

Der Festkörper wurde einmal mit deionisiertem Wasser und einmal mit einem 94:6 Gemisch (v/v) von Ethanol und deionisiertem Wasser gewaschen. Beide Waschlösungen hatten eine Temperatur von 5°C bis 10°C.

Der gewaschene, amorphe Festkörper wurde unter reduziertem Druck (20 bis 30 mbar) bei Raumtemperatur während 2 Stunden getrocknet.

Es wurden 146 g amorphe (6S)-Folinsäure erhalten.
HPLC-Reinheit: 99,2 %
HPLC-Assay/Gehalt: 99,0 %

Die Diastereoisomerenreinheit betrug 99,7 % (HPLC).

Dieser Festkörper wurde bei einer Temperatur von 2°C bis 8°C in einem Kühlschrank gelagert. Dabei wurden für diesen Festkörper die folgenden Stabilitätsdaten erhalten:

| Zeit (Monate) | Assay (HPLC) | Reinheit (HPLC) |
|---|---|---|
| 0 | 99, 0% | 99, 2% |
| 1 | 98, 5% | 99, 0% |
| 2 | 98, 1% | 98, 9% |
| 3 | 98, 9% | 98, 9% |
| 6 | 98, 1% | 98, 4% |
| 12 | 97, 8% | 97, 2%. |

### Beispiel 3

100 g Amorphe (6S)-Folinsäure, hergestellt gemäss Beispiel 2, wurden in 1,2 Liter entgastem, sterilem Wasser unter einer Stickstoffatmosphäre bei Raumtemperatur suspendiert.

Anschliessend wurde unter Rühren eine 10%-ige wässrige Natriumhydroxidlösung hinzugetropft, und zwar so lange, bis eine klare Lösung entstanden war, welche einen pH-Wert von 8,0 hatte.

Die erhaltene klare Lösung wurde durch Hinzugabe von entgastem, sterilem Wasser auf ein Volumen von 1,8 Liter verdünnt.

Diese verdünnte Lösung wurde einer sterilen Filtration (Porengrösse: 0,2 Mikrometer) unterworfen.

Das erhaltene sterile Filtrat wurde unter Stickstoffatmosphäre in 10 ml fassende Glasvials abgefüllt.

Diese Glasvials wurden bei einer Temperatur von 2°C bis 8°C in einem Kühlschrank gelagert. Dabei wurden für die in die Glasvials abgefüllte Lösung die folgenden Stabilitätsdaten erhalten:

| Zeit (Monate) | Assay (HPLC) | Reinheit (HPLC) |
|---|---|---|
| 0 | 100, 0% | 98, 4% |
| 1 | 101, 0% | 98, 4% |
| 3 | 106, 0% | 98, 3% |

| Zeit (Monate) | Assay (HPLC) | Reinheit (HPLC) |
|---|---|---|
| 6 | 110, 9% | 98, 9% |
| 9 | 108, 0% | 98, 1% |
| 12 | 111, 7% | 97, 6%. |

Nach 12 Monaten war die Lösung immer noch klar; Niederschläge konnten keine festgestellt werden.

## Patentansprüche

1. Verfahren zur Herstellung einer konzentrierten, stabilen Lösung, insbesondere einer Injektionslösung oder Infusionslösung, des Natrium- oder Kaliumsalzes von (6S)-Folinsäure,
wobei man amorphe (6S)-Folinsäure in Wasser, welches entgast und für die Herstellung von Injektionslösungen oder Infusionslösungen annehmbar ist, bei Raumtemperatur unter einer Inertgasatmosphäre suspendiert, dann
eine wässrige Lösung von Natrium- oder Kaliumhydroxid, -hydrogencarbonat oder -carbonat portionenweise so lange hinzugibt, bis eine klare Lösung entstanden ist, welche den jeweils gewünschten pH-Wert aufweist,
die erhaltene Lösung einer sterilen Filtration unterwirft, und
die erhaltende sterile Lösung unter einer Inertgasatmosphäre in Vials oder in Ampullen abfüllt,
**dadurch gekennzeichnet, dass**
die amorphe (6S)-Folinsäure gemäss einem Verfahren hergestellt wird, bei dem man
zu gerührtem Wasser, welches eine Temperatur von 2°C bis 12°C hat, gleichzeitig
- eine wässrige Lösung, welche eine Temperatur von 40°C bis 50°C hat, von (6S)-Calcium-Folinat, und
- eine wässrige Lösung von Salzsäure oder Essigsäure
derart hinzugibt, dass im erhaltenen Gemisch während der Hinzugabe der beiden genannten Lösungen einerseits die Temperatur auf einem Wert von 2°C bis 12°C gehalten wird, und ahdererseits der pH-Wert auf einem Wert von 2,5 bis 3,5 gehalten wird,
den entstandenen Festkörper mittels Filtration oder Zentrifugation isoliert,
diesen Festkörper zuerst mit kaltem Wasser und dann mit einem wässrigen organischen Lösungsmittel wäscht, und
den gewaschenen Festkörper, nämlich amorphe (6S)-N(5)-Formyl-5,6,7,8-tetrahydrofolsäure, unter reduzierten Druck trocknet und gewinnt.

2. Verfahren nach Anspruch 1, **dadurch gekennzeichnet, dass** das gerührte Wasser, zu welchem die beiden genannten Lösungen gleichzeitig hinzugegeben werden, eine Temperatur von 6°C bis 10°C hat.

3. Verfahren nach einem der Ansprüche 1 bis 2, **dadurch gekennzeichnet, dass** die wässrige Lösung von (6S)-Calcium-Folinat eine Konzentration von 3,0 Gew.-% bis 3,7 Gew.-%, vorzugsweise 3,5 Gew.-%, hat.

4. Verfahren nach einem der Ansprüche 1 bis 3, **dadurch gekennzeichnet, dass** die wässrige Lösung von (6S)-Calcium-Folinat eine Temperatur von 46°C hat.

5. Verfahren nach einem der Ansprüche 1 bis 4, **dadurch gekennzeichnet, dass** die wässrige Lösung von Salzsäure Raumtemperatur aufweist und eine Konzentration von 10 Gew.-% bis 20 Gew.-%, vorzugsweise 18 Gew.-%, hat.

6. Verfahren nach einem der Ansprüche 1 bis 5, **dadurch gekennzeichnet, dass** im erhaltenen Gemisch während der gleichzeitigen Hinzugabe der beiden genannten Lösungen die Temperatur auf einem Wert von 6°C bis 10°C gehalten wird.

7. Verfahren nach einem der Ansprüche 1 bis 6, **dadurch gekennzeichnet, dass** im erhaltenen Gemisch während der gleichzeitigen Hinzugabe der beiden genannten Lösungen der pH-Wert auf einem Wert von 2,8 bis 3,2 gehalten wird.

8. Verfahren nach einem der Ansprüche 1 bis 7, **dadurch gekennzeichnet, dass** nach erfolgter gleichzeitiger Hinzugabe der beiden genannten Lösungen das erhaltene Gemisch noch 1 Stunde bei einer Temperatur von 6°C bis 10°C weiter gerührt wird.

9. Verfahren nach einem der Ansprüche 1 bis 8, **dadurch gekennzeichnet, dass** der entstandene amorphe Festkörper nach dem Waschen mit kaltem Wasser mit einem 94:6 Gemisch (v/v) von Ethanol und Wasser gewaschen wird.

10. Verfahren nach Anspruch 1, **dadurch gekennzeichnet, dass** die genannte klare Lösung von 2 Gew.-% bis 15 Gew.-%, insbesondere von 2 Gew.-% bis 6 Gew.-%, vorzugsweise 5 Gew.-%, (6S)-Natrium-Folinat oder (6S)-Kalium-Folinat enthält.

11. Verfahren nach einem der Ansprüche 1 oder 10, **dadurch gekennzeichnet, dass** die genannte klare Lösung einen pH-Wert im Bereich von 7,5 bis 8,5, insbesondere 7,9 bis 8,1, vorzugsweise 8,0, aufweist.

12. Amorphe (6S)-N(5)-Formyl-5,6,7,8-tetra-hydrofolsäure, **dadurch gekennzeichnet, dass** diese Verbindung nach dem Verfahren gemäss einem der Ansprüche 1 bis 9 hergestellt ist.

13. Verwendung von amorpher (6S)-N(5)-Formyl-5,6,7,8-tetrahydrofolsäure, hergestellt gemäss dem Verfahren nach einem der Ansprüche 1 bis 9, zur Herstellung einer wässrigen Lösung des Natrium- oder Kaliumsalzes von (6S)-Folinsäure.

## Claims

1. A process for the preparation of a concentrated, stable solution, especially of an injection solution or of an infusion solution, of the sodium or potassium salt of (6S)-folinic acid,
whereby amorphous (6S)-folinic acid is suspended in water, that is degassed and that is acceptable for the preparation of injection solutions or of infusion solutions, at room temperature under an inert gas atmosphere, then
an aqueous solution of sodium or potassium hydroxide, -hydrogencarbonate or -carbonate is added in portions during such a long time until a clear solution is formed having the respective desired pH value,
the obtained solution is subjected to a sterile filtration, and
the obtained sterile solution is filled into vials or into ampoules under an inert gas atmosphere,
**characterized in that** the amorphous (6S)-folinic acid is prepared according to a process **in that**
there is added to stirred water having a temperature from 2°C to 12°C simultaneously
- an aqueous solution having a temperature from 40°C to 50°C of (6S)-calcium-folinate, and
- an aqueous solution of hydrochloric acid or of acetic acid
in such a way that in the obtained mixture during the addition of both of said solutions on one hand the temperature is kept at a value from 2°C to 12°C and on the other hand the pH value is kept at a value from 2.5 to 3.5,
the formed solid is isolated by means of filtration or centrifugation,
this solid is washed first with cold water and then with an aqueous organic solvent, and
the washed solid, that is amorphous (GS)-N(5)-formyl-5,6,7,8-tetrahydrofolic acid, is dried under reduced pressure and is obtained.

2. The process according to claim 1, **characterized in that** the stirred water, to which said two solutions are added simultaneously, has a temperature from 6°C to 10°C.

3. The process according to one of claims 1 to 2, **characterized in that** the aqueous solution of (6S)-calcium-folinate has a concentration from 3.0 % by weight to 3.7 % by weight, preferably 3.5 % by weight.

4. The process according to one of claims 1 to 3, **characterized in that** the aqueous solution of (6S)-calcium-folinate has a temperature of 46°C.

5. The process according to one of claims 1 to 4, **characterized in that** the aqueous solution of hydrochloric acid has room temperature and has a concentration from 10 % by weight to 20 % by weight, preferably 18 % by weight.

6. The process according to one of claims 1 to 5, **characterized in that** in the obtained mixture during the simultaneous addition of both of said solutions the temperature is kept at a value from 6°C to 10°C.

7. The process according to one of claims 1 to 6, **characterized in that** in the obtained mixture during the simultaneous addition of both of said solutions the pH value is kept at a value from 2.8 to 3.2.

8. The process according to one of claims 1 to 7, **characterized in that** after the realized simultaneous addition of both of said solutions the obtained mixture is stirred for 1 additional hour at a temperature from 6°C to 10°C.

9. The process according to one of claims 1 to 8, **characterized in that** the formed amorphous solid is washed after the washing with cold water with a 94:6 mixture (v/v) of ethanol and water.

10. The process according to claim 1, **characterized in that** said clear solution contains from 2 % by weight to 15 % by weight, especially from 2 % by weight to 6 % by weight, preferably 5 % by weight, of (6S)-sodium-folinate or of (6S)-potassium-folinate.

11. The according to one of claims 1 or 10, **characterized in that** said clear solution has a pH value in the range from 7.5 to 8.5, especially from 7.9 to 8.1, preferably 8.0.

12. Amorphous (6S)-N(5)-formyl-5,6,7,8-tetrahydrofolic acid, **characterized in that** this compound has been prepared according to the process according to one of claims 1 to 9.

13. Use of amorphous (6S)-N(5)-formyl-5,6,7,8-tetrahydrofolic acid, prepared according to the process according to one of claims 1 to 9, for the preparation of an aqueous solution of the sodium or potassium salt of (6S)-folinic acid.

## Revendications

1. Procédé de préparation d'une solution concentrée stable, en particulier d'une solution pour injection ou perfusion, du sel de sodium ou de potassium de l'acide (6S)-folinique, dans lequel
on met en suspension de l'acide (6S)-folinique amorphe dans de l'eau, qui est dégazée et acceptable pour la préparation de solutions pour injection ou perfusion, à température ambiante et sous une atmosphère de gaz inerte,
on ajoute ensuite une solution aqueuse d'hydroxyde, d'hydrogénocarbonate ou de carbonate de sodium ou de potassium, par portions, jusqu'à obtention d'une solution limpide, qui présente le pH souhaité dans chaque cas,
on soumet la solution obtenue à une filtration stérile, et
on transvase la solution stérile obtenue dans des fioles ou des ampoules, sous une atmosphère de gaz inerte,
**caractérisé en ce que**
l'acide (6S)-folinique amorphe est produit selon un procédé dans lequel
on ajoute à de l'eau sous agitation, qui présente une température comprise entre 2 °C et 12 °C, simultanément
- une solution aqueuse de (6S)-folinate de calcium, qui présente une température comprise entre 40 °C et 50 °C et
- une solution aqueuse d'acide chlorhydrique ou d'acide acétique
de manière telle que dans le mélange obtenu, au cours de l'addition des deux solutions citées, d'une part, la température est maintenue à une valeur comprise entre 2 °C et 12 °C et, d'autre part, le pH est maintenu à une valeur comprise entre 2,5 et 3,5,
on isole le solide formé par filtration ou centrifugation,
on lave ce solide tout d'abord à l'eau froide et ensuite avec un solvant organique aqueux, et
on sèche et on extrait sous pression réduite le solide lavé, à savoir l'acide (6S)-N-(5)-formyl-5,6,7,8-tétrahydrofolinique amorphe.

2. Procédé selon la revendication 1, **caractérisé en ce que** l'eau sous agitation à laquelle les deux solutions citées sont ajoutées simultanément, présente une température comprise entre 6 °C et 10 °C.

3. Procédé selon l'une des revendications 1 à 2, **caractérisé en ce que** la solution aqueuse de (6S)-folinate de calcium présente une concentration comprise entre 3,0 et 3,7 % en poids, de préférence de 3,5 % en poids.

4. Procédé selon l'une des revendications 1 à 3, **caractérisé en ce que** la solution aqueuse de (6S)-folinate de calcium présente une température de 46 °C.

5. Procédé selon l'une des revendications 1 à 4, **caractérisé en ce que** la solution aqueuse d'acide chlorhydrique est à température ambiante et présente une concentration comprise entre 10 et 20 % en poids, de préférence de 18 % en poids.

6. Procédé selon l'une des revendications 1 à 5, **caractérisé en ce que** dans le mélange obtenu, au cours de l'addition simultanée des deux solutions citées, la température est maintenue à une valeur comprise entre 6 °C et 10 °C.

7. Procédé selon l'une des revendications 1 à 6, **caractérisé en ce que** dans le mélange obtenu, au cours de l'addition simultanée des deux solutions citées, le pH est maintenu à une valeur comprise entre 2,8 et 3,2.

8. Procédé selon l'une des revendications 1 à 7, **caractérisé en ce qu'**après l'addition simultanée des deux solutions citées, le mélange obtenu est encore agité pendant 1 heure à une température comprise entre 6 °C et 10 °C.

9. Procédé selon l'une des revendications 1 à 8, **caractérisé en ce qu'**après avoir été lavé à l'eau froide, le solide amorphe obtenu est lavé avec un mélange 94:6 (v/v) d'éthanol et d'eau.

10. Procédé selon la revendication 1, **caractérisé en ce que** la solution limpide citée contient de 2 à 15 % en poids, en particulier de 2 à 6 % en poids, de préférence 5 % en poids de (6S)-folinate de sodium ou de (6S)-folinate de potassium.

11. Procédé selon l'une des revendications 1 ou 10, caractérisé en que la solution limpide citée présente une valeur de pH comprise dans l'intervalle de 7,5 à 8,5, en particulier de 7,9 à 8,1, de préférence de 8,0.

12. Acide (6S)-N-(5)-formyl-5,6,7,8-tétrahydrofolinique amorphe, **caractérisé en ce que** ce composé est produit selon le procédé conforme à l'une des revendications 1 à 9.

13. Utilisation de l'acide (6S)-N-(5)-formyl-5,6,7,8-tétrahydrofolinique amorphe, produit selon le procédé conforme à l'une des revendications 1 à 9, pour la préparation d'une solution aqueuse du sel de sodium ou de potassium de l'acide (6S)-folinique.
